(19) Europäisches Patentamt
European Patent Office
Office européen des brevets

(11) Publication number: **0 248 765**
**A2**

# (12) EUROPEAN PATENT APPLICATION

(21) Application number: **87810315.9**

(22) Date of filing: **27.05.87**

(51) Int. Cl.[4]: **C 07 D 231/40**
C 07 D 231/50, A 01 N 43/56

(30) Priority: **06.06.86 US 871234**

(43) Date of publication of application:
**09.12.87 Bulletin 87/50**

(84) Designated Contracting States:
**AT BE CH DE ES FR GB GR IT LI LU NL SE**

(71) Applicant: **SANDOZ AG**
**Lichtstrasse 35**
**CH-4002 Basel (CH)**

(84) Designated Contracting States:
**BE CH ES FR GB GR IT LI LU NL SE**

(71) Applicant: **SANDOZ-PATENT-GMBH**
**Humboldtstrasse 3**
**D-7850 Lörrach (DE)**

(84) Designated Contracting States: **DE**

(71) Applicant: **SANDOZ-ERFINDUNGEN**
**Verwaltungsgesellschaft m.b.H.**
**Brunner Strasse 59**
**A-1235 Wien (AT)**

(84) Designated Contracting States: **AT**

(72) Inventor: **Carney, Robert L.**
**2676 Emerson Street**
**Palo Alto, CA 94306 (US)**

**Gruber, John M.**
**565 Willow Road Apt. 4**
**Menlo Park CA 94025 (US)**

**Lui, Alfred S.T.**
**716 Windsor Way**
**Redwood City, CA 94062 (US)**

Claims for the following Contracting States: AT + ES.

(54) **Novel benzoylureas.**

(57) Novel compounds of formula (A)

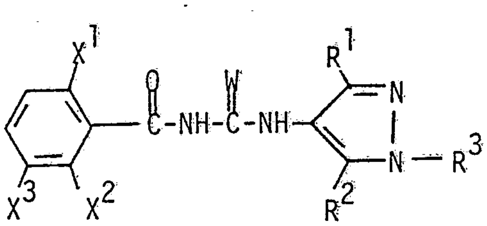

(A)

wherein W, $X^1$, $X^2$, $X^3$, $R^1$, $R^2$ and $R^3$ are as specified in the description, the preparation of such compounds, their use for the control of pests and pest-controlling compositions comprising such compounds.

EP 0 248 765 A2

## Description

### NOVEL BENZOYLUREA

The present invention relates to novel substituted N-(4-pyrazolyl)-N'-benzoylureas, to processes for producing these compounds, to the use of the compounds for the control of pests and to pest-controlling compositions comprising such compounds.

The compounds of the present invention are represented by the following formula (A)

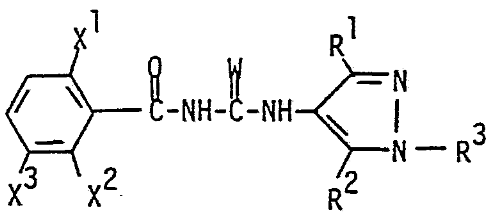

(A)

wherein W is oxygen or sulfur;

each of $X^1$, $X^2$ and $X^3$ is independently hydrogen, $C_{1-8}$alkyl or halogen,

each of $R^1$ and $R^2$ is independently hydrogen, $C_{1-8}$alkyl, $C_{1-8}$alkoxy, halogen or COOR;

R is hydrogen or $C_{1-8}$alkyl;

$R^3$ is unsubstituted or halogenated $C_{1-8}$alkyl, unsubstituted or halogenated $C_{2-8}$alkenyl, unsubstituted or halogenated $C_{3-8}$cycloalkyl, or the substituted phenyl group

$C_6H_{4-t}ZY_t$

wherein t is zero, one, two, three or four; and

each of Y and Z is independently halogen or unsubstituted or halogenated $C_{1-4}$alkyl.

Where any of the substituents $X^1$-$X^3$, $R^1$-$R^3$, Y and Z is or comprises halogen, such halogen is conveniently selected from bromo, chloro or fluoro.

Where any of $X^1$-$X^3$ is $C_{1-8}$alkyl, it is preferably of l to 4 carbon atoms and is more preferably of l or 2 carbons.

Where any of R-$R^3$ is $C_{1-8}$alkyl, it is preferably of l to 4 carbon atoms.

Where either of $R^1$ or $R^2$ is $C_{1-8}$alkoxy, it is preferably of l to 4 carbons and is more preferably of l or 2 carbons.

Where $R^3$ is $C_{3-8}$cycloalkyl, it is preferably of 3 to 6 carbon atoms.

Where $R^3$ is $C_{2-8}$alkenyl, it is preferably of 2 to 4 carbon atoms.

The terms halogenated $C_{1-8}$alkyl and halogenated $C_{3-8}$cycloalkyl refer to $C_{1-8}$alkyl and $C_{3-8}$cycloalkyl, respectively, substituted by l to 8, preferably l to 6, and more preferably l to 3 halogens; such halogen is preferably chloro or fluoro.and more preferably fluoro.

The term halogenated $C_{2-8}$alkenyl refers to a $C_{2-8}$alkenyl substituted by l to 7, preferably l to 6 halogens; such halogen is preferably chloro or fluoro.

In the practice of the present invention, W is preferably oxygen.

$X^1$ is preferably halogen; such halogen is preferably chloro or fluoro, more preferably fluoro.

$X^2$ is preferably hydrogen or halogen, more preferably halogen; such halogen is preferably fluoro.

$X^3$ is preferably hydrogen.

$R^1$ conveniently signifies hydrogen, $C_{1-4}$alkyl, $C_{1-4}$alkoxy or halogen; it is preferably hydrogen, methyl, methoxy or chloro.

$R^2$ conveniently signifies hydrogen, $C_{1-4}$alkyl, $C_{1-4}$alkoxy or halogen; it is preferably hydrogen, methyl or chloro.

$R^3$ conveniently signifies unsubstituted or halogenated $C_{1-4}$alkyl or the substituted phenyl group; it is preferably the substituted phenyl group.

t is preferably one or two, more preferably one.

Y is preferably halogen; such halogen is preferably chloro or fluoro, more preferably chloro.

Z is preferably halogen substituted $C_{1-4}$alkyl or halogen; such halogen is preferably chloro or fluoro. Z is more preferably $CF_3$ or chloro.

The compounds of the present invention of formula (A) are obtained-by

a) reacting a benzoyl isocyanate or isothiocyanate of formula (I)

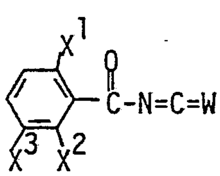

(I)

wherein $X^1$, $X^2$, $X^3$ and W are as defined above with a 4-aminopyrazole of formula (II)

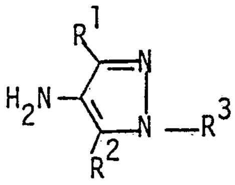

(II)

wherein $R^1$, $R^2$ and $R^3$ are as defined above; or

b) by reacting a benzamide of formula (III)

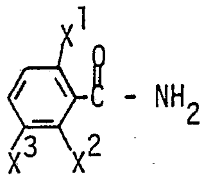

(III)

wherein $X^1$, $X^2$ and $X^3$ are as defined above, with a pyrazolyl isocyanate or isothiocyanate of formula (IV)

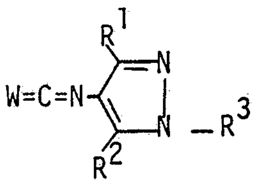

(IV)

wherein W, $R^1$, $R^2$ and $R^3$ are as defined above.

The reaction of compounds of formula I with compounds of formula II (process a) may be effected under the conditions known for the preparation of N-benzoylureas from the corresponding isocyanates and amines.

The reaction is conveniently carried out in a solvent which is inert under the reaction conditions, e.g. methylene chloride, toluene or dimethylformamide. A suitable reaction temperature may vary from -10° C to the boiling point of the solvent used, and preferably is about room temperature or moderately above or below room temperature, e.g. between 15 and 25° C. The reaction is optionally carried out in the presence of an organic base, for example triethylamine.

The reaction of compounds of formula III with compounds of formula IV (process b) may be effected under the conditions known for the preparation of N-benzoylureas from the corresponding benzamides and isocyanates or isothiocyanates.

The reaction is conveniently carried out in a solvent which is inert under the reaction conditions and at a reaction temperature of between 0° and 120° C, preferably at the boiling point of the solvent used, and optionally in the presence of an organic base, such as pyridine.

The compounds of formula (A) may be recovered from the reaction mixture in which they are formed by working up by established procedures.

The starting materials and reagents employed in the processes described above are either known or, in cases where they are novel, may be produced by methods analogous to the processes described herein or to known processes.

The compounds of formula (A) can have one or more asymmetric certers and/or geometric isomers. The present invention includes each of the stereo isomers and the mixtures of stereo isomers thereof. In the examples hereinafter, unless otherwise specified, the compounds having such asymmetric center(s) and/or geometric isomers are obtained as a mixture of stereo isomers.

The compounds of the present invention of formula (A) are useful pest control agents, particularly for the control of insects, mites and ticks. These compounds can be effective control agents for insects of, for example, the orders Lepidoptera, Hemiptera, Homoptera, Coleoptera, Diptera, Orthoptera and Siphonaptera, and other insects, as well as for mites and ticks of the class Acari, including mites of the families Tetranychidae and Tarsonemidae and ticks of the families Argasidae and Ixodidae. The compounds can be applied to the pest or its locus in a pest-controlling amount, usually of the order of 0.001 microgram to 100 microgram per insect, mite or tick, depending on the mode and conditions of application as well as on the pest involved.

Additionally, compounds of formula A may possess a repellent and/or antifeedant action on terrestrial snails and slugs. The compounds may be useful for control of arthropod parasites of vertebrates, either by topical

application or by oral administration.

In the use of the compounds of formula (A) for combatting pests, a compound of formula (A), or mixtures thereof, can conveniently be employed as pesticidal compositions in association with acceptable diluent(s) for application to the pest or its locus. Such compositions also form part of the present invention.

Suitable formulations contain from O.Ol to 99% by weight of active ingredient, from O to 2O% of surfactant and from l to 99.99% of solid or liquid diluent(s). Higher ratios of surfactant to active ingredient are sometimes desirable and are achieved by incorporation into the formulation or by tank mixing. Application forms of a composition generally contain between O.Ol and 25% by weight of active ingredient. Lower or higher levels of active ingredient can, of course, be present depending on the intended use, the physical properties of the compound and the mode of application. Concentrate forms of a compositions intended to be diluted before use generally contain between 2 and 9O%, preferably between 5 and 85% by weight of active ingredients.

Useful formulations of the compounds of formula (A) include dusts, granules, suspension concentrates, wettable powders, flowables and the like. They are obtained by conventional manner, e.g. by mixing a compound of formula (A) with the diluent(s) and optionally with other ingredients.

Alternatively, the compounds of formula (A) may be used in microencapsulated form.

The compounds of formula (A) can be combined with cyclodextrin to make a cyclodextrin inclusion complex for application to the pest or its locus.

Agriculturally acceptable additives may be employed in the pesticidal compositions to improve the performance of the active ingredient and to reduce foaming, caking and corrosion, for example.

"Surfactant" as used herein means an agriculturally acceptable material which imparts emulsifiability, spreading, wetting, dispersibility or other surface-modifying properties. Examples of surfactants are sodium lignin sulfonate and lauryl sulfate.

"Diluent" as used herein means a liquid or solid agriculturally acceptable material used to dilute a concentrated material to a usable or desirable strength. For dusts or granules it can bee.g. talc, kaolin or diatomaceous earth, for liquid concentrate forms for example a hydrocarbon such as xylene or an alcohol such as isopropanol, and for liquid application forms i.e. water or diesel oil.

The compositions of this invention can also comprise other compounds having biological activity, e.g. compounds having similar or complementary pesticidal or insect growth regulating activity or compounds having antidotal, fungicidal, herbicidal or insect attractant activity.

The following examples are provided to illustrate the practice of the present invention. Temperature is given in degrees Centigrade. RT means for temperature. Parts and percentages are by weight.

COMPOSITION EXAMPLES

Example A: Flowable

| | |
|---|---|
| Compound 1 | 26.00% |
| Dispersant | 5.20% |
| Thickener | 0.58% |
| Propylene glycol | 6.60% |
| Antifoam | 0.10% |
| Water | 61.52% |

Example B: Wettable Powder

| | |
|---|---|
| Compound 1 | 81.0% |
| kaolin | 14.8% |
| dispersant | 4.0% |
| wetting agent | 0.2% |

PREPARATION OF FINAL UREAS

Example l: N-4-[l-(2-chloro-4-trifluoromethylphenyl)pyrazolyl]-N′-2,6 difluorobenzoylurea

4-Amino-l-(2-chloro-4-trifluoromethylphenyl)pyrazole (O.5 g, 3.8 mmol) is dissolved in 5 ml ofdimethylformamide (DMF), after which 7 ml of methylene chloride is added. 2,6-Difluoro-benzoyl isocyanate (O.7 g, 3.8 mmol) is added to the solution, and the reaction mixture is stirred at RT overnight. It is then poured into water and

filtered, and the resulting white solid is washed with ether and air dried to give N-4-[2-chloro-4-trifluoromethyl-phenyl)pyrazolyl]-N'-2,6-difluorobenzoylurea, (compound I, Table A).

Example 2:

Following the procedures of Example I, each of the final compounds 2-2I under Table A is prepared from the corresponding pyrazole and benzoyl isocyanate or isothiocyanate intermediates.

Example 3:

4-Amino-I-(I,I,2,3,3,3-hexafluoropropyl)pyrazole is reacted with each of 2,6-difluorobenzoyl isocyanate and 2-chlorobenzoyl isocyanate, following the procedure of Example I, to give, respectively,

N-4-[I-(I,I,2,3,3,3-hexafluoropropyl)pyrazolyl]-N'-2,6-difluorobenzoylurea, m.p. I75-I77° (compound 22) and N-4-[I-(I,I,2,3,3,3-hexafluoropropyl)pyrazolyl]-N'-2-chlorobenzoylurea (compound 23).

BIOLOGICAL ACTIVITY:

Example 4:

Early (O-24 hr) third instar larvae of the tobacco budworm, Heliothis virescens, are topically treated on the dorsal abdomen with I ul of acetone dilution of the rest compound at the concentration to be tested. The treated larvae are placed on artificial diet in individual cells of a plastic grid contained in a covered plastic petri dish. The containers are held at 27° C, I6 hour photoperiod until all larvae are either dead or have molted to fifth instar larvae. The compounds of formula (A) demonstrate insecticidal activity. Compound I has for example an $ED_{50}$ of O.O79 microgram per larva in this test ($ED_{50}$ is the dose at which 5O % of the test insects are affected by the compound).

Example 5:

Mature third instar larvae (wandering stage having left food) of the housefly, Musca domestica, are topically treated with I ul of acetone dilution of the test compound at the concentration to be tested. The treated larvae are allowed to pupate and are held at 3I° C, I6 hour photoperiod until all insects have died or emerged from pupae adults (about 7 days). The compounds of formula (A) demonstrate insecticidal activity.

Example 6:

Early (O-24 hr) third instar larvae of the beet armyworm, Spodoptera exigua, are topically treated on the dorsal abdomen with I ul of acetone dilution of the test compound at the concentration to be tested. The treated larvae are placed on artificial diet in a covered plastic petri dish. The containers are held at 27° C, I6 hour photoperiod until all larvae are either dead or have pupated. The compounds of formula (A) demonstrate insecticidal activity. The $ED_{50}$ of compound I is O.OI7 microgram per larva.

TABLE A

Compounds of formula (A) wherein $X^3$ is H, W is O and $R^3$ is $C_6H_{4-t}ZY_t$

| Cpd | $X^1$ | $X^2$ | W | $R^1$ | $R^2$ | $(Y)_t$ | Z | m.p. (°C) |
|---|---|---|---|---|---|---|---|---|
| 1 | F | F | O | H | H | 2-Cl | 4-$CF_3$ | 222-224 |
| 2 | F | F | O | H | H | 2,6-Cl | 4-$CF_3$ | 212-213 |
| 3 | F | F | O | H | H | 4-Cl | 3-$CF_3$ | 213-215 |
| 4 | F | F | O | H | H | 2-Cl | 4-Cl | 201-202 |
| 5 | F | F | O | H | H | 3-Cl | 5-Cl | 235-236.5 |
| 6 | F | F | O | H | H | 2,3,4,5-Cl | 6-Cl | |
| 7 | F | F | O | Cl | H | 2-Cl | 4-$CF_3$ | 219-220 |
| 8 | F | F | O | Cl | Cl | 2-Cl | 4-$CF_3$ | 206.5-207 |
| 9 | F | F | O | $OCH_3$ | Cl | 2-Cl | 4-$CF_3$ | 197-198 |
| 10 | F | F | O | $CH_3$ | H | 2-Cl | 4-$CF_3$ | 206-207.5 |
| 11 | F | F | O | H | $CH_3$ | 2-Cl | 4-$CF_3$ | 194-195 |
| 12 | F | F | O | $CH_3$ | $CH_3$ | 2-Cl | 4-$CF_3$ | 201-203 |
| 13 | F | F | S | H | H | 2-Cl | 4-$CF_3$ | 198-199 |
| 14 | H | H | S | H | H | 2-Cl | 4-$CF_3$ | 175-176 |
| 15 | Cl | H | O | H | H | 2-Cl | 4-$CF_3$ | 209-211 |
| 16 | Cl | H | O | H | H | 2,6-Cl | 4-$CF_3$ | |
| 17 | Cl | H | O | H | H | 4-Cl | 3-$CF_3$ | 189-190 |
| 18 | Cl | H | O | H | H | 2-Cl | 4-Cl | |
| 19 | Cl | H | O | H | H | 3-Cl | 5-Cl | |
| 20 | $CH_3$ | H | O | H | H | 2-Cl | 4-$CF_3$ | 216-217 |
| 21 | Cl | H | O | $CH_3$ | H | 2-Cl | 4-$CF_3$ | 206-207 |

The starting materials of formulae I, II, III and IV herein are known or, in cases where they are novel, may be produced by methods analogous to known methods or by methods described herein.

Thus, the compounds of formula I can be synthesized by a) treating the corresponding benzamide with oxalyl chloride in the presence of a solvent such as chlorinated hydrocarbon, or b) reacting the corresponding benzoyl chloride with ammonium thioxyanate.

The aminopyrazole derivatives of formula II can be prepared by reduction of catalytic hydrogenation of the corresponding nitro compounds of formula (V).

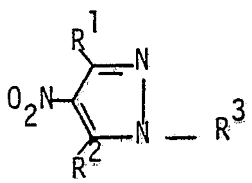

(V)

The isocyanates and isothiocyanates of formula (IV) can be produced by reaction of the aminopyrazole derivatives of formula (II) with phosgene or thiophosgene by use of customary procedures.

The nitro compounds of formula (V) can be synthesized by reacting a pyrazole (VI) with nitric acid in sulfuric acid or acetic anhydride.

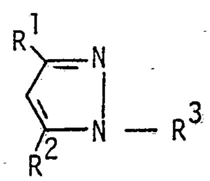

(VI)

Alternatively, the nitro compounds (V) may be prepared by reacting the unsubstituted nitropyrazole (V; where $R^3$ is hydrogen) with sodium hydride in a solvent such as tetrahydrofuran, dimethylformamide, hexamethylphosphoramide or N-methylpyrrolidinone, and the resulting sodium salt is reacted with $R^3$-X (where X is a halogen) to give (V).

Where $R^3$ is a haloalkyl group of the formula $FCHR^4$-$CHR^5R^6$ (wherein each of $R^4$, $R^5$ and $R^6$ is independently halogen or lower haloalkyl and $R^4$ and $R^5$ may optionally comprise aring-forming haloalkyl chain of two to four carbon atoms), the nitro compound (V) may be prepared by reacting the unsubstituted 4-nitropyrazole (V, where $R^3$ is hydrogen) with the olefin $FCR^4=CR^5R^6$ in the presence of a base catalyst such as sodium hydroxide.

Where $R^3$ is a haloalkenyl group of the formula $FCR^4=CR^5R^6$ (wherein $R^4$, $R^5$ and $R^6$ are as defined above), the nitro compound (V) may be prepared by reacting the unsubstituted 4-nitropyrazole ((V), where $R^3$ is hydrogen) with sodium hydride in a suitable aprotic solvent such as dimethylformamide, hexamethylphosphoramide or N-methylpyrrolidinone and then reacting the resulting sodium salt with an olefin of the formula $FCR^4=CR^5R^6$.

Alternatively, compounds of formula (V) where $R^3$ is substituted phenyl can be prepared by reacting together a phenylhydrazine and I,I,3,3-tetramethoxypropane and an acid such as sulfuric acid in a solvent such as methanol or ethanol and at an elevated temperature, and treating the resulting phenyl-substituted pyrazole with nitric acid, by the method outlined above.

INTERMEDIATE COMPOUNDS

The following examples are presented to illustrate representative methods of preparing the intermediate amino compounds.

Example 7: 4-Amino-I-(2-chloro-4-trifluoromethylphenyl)-pyrazole

To 24 ml of fuming conc. sulfuric acid cooled in an ice bath is added pyrazole (I2.O g, I76.O mmol), in small portions. In a separate flask is charged 24 ml of fuming conc. sulfuric acid cooled to 5°, and 24 ml of fuming nitric acid is added dropwise. After addition is complete, the nitric acid/sulfuric acid mixture is added dropwise to the cooled pyrazole. After addition is complete, the rection mixture is heated to IIO°C for 5O hours. It is then cooled and poured into ice, and the resulting white solid is filtered, washed with water and air dried to give 4-nitropyrazole.

A solution of the above 4-nitropyrazole (I.5 g, I3.3 mmol) in IO ml of tetrahydrofuran (THF) is added to a suspension of 5O% sodium hydride (O.64 g, I3.3 mmol) in IO ml of THF and 5 ml of hexamethylphosphoramide (HMPA). The mixture is stirred at RT for I hour, after which a solution of 3-chloro-4-fluorobenzotrifluoride (2.4 g, I3.3 mmol) in 2O ml of THF is added. The reaction mixture is stirred overnight at RT and is then heated to

60°C for 3 hours. It is poured into water and extracted with ether, and the organic extracts are washed with water and with brine, and dried to give, after purification, 4-nitro-1-(2-chloro-4-trifluoromethylphenyl)pyrazole.

A mixture of 4-nitro-1-(2-chloro-4-trifluoromethylphenyl)-pyrazole (1.6 g, 5.5 mmol) and platinum IV oxide (0.04 g) in 20 ml of ethanol and 10 ml of ethyl acetate is stirred under $H_2$ at RT and atmospheric pressure. After hydrogen uptake is complete, the catalyst is filtered off and solvent is stripped off to give 4-amino-1-(2-chloro-4-trifluoromethylphenyl)-pyrazole.

Example 8: 4-Amino-1-(2,6-dichloro-4-trifluoromethylphenyl)-pyrazole

A mixture of 3,5-dichloro-4-fluorobenzotrifluoride (1.17 g, 5.0 mmol), pyrazole (0.30 g, 5.5 mmol) and potassium carbonate (0.69 g, 5.0 mmol) in 5 ml of dimethylformamide (DMF) is heated to 110°C for 1.5 hours. It is then cooled and poured into ether/hexane, washed with water, dried and concentrated to give, after purification by flash chromatography 1-(2,6-dichloro-4-trifluoromethylphenyl)pyrazole.

The above phenylpyrazole is dissolved in 8 ml of sulfuric acid to which is added 3 ml of fuming nitric acid and then 1.5 ml of fuming sulfuric acid. The reaction mixture is stirred overnight and is then poured into ether/hexane and ice water, washed with water and with sodium bicarbonate, dried, concentrated and purified by flash chromatography to give 4-nitro-1-(2,6-dichloro-4-trifluoromethylphenyl)pyrazole, a white solid.

To a solution of the above nitropyrazole (0.64 g, 1.96 mmol) in 20 ml of ethanol is added platinum dioxide (0.05 g), and the mixture is stirred under an $H_2$ atmosphere for 2 hours. The crude product is filtered, partitioned into ether/sodium bicarbonate, washed with brine, dried, concentrated and purified by flash chromatography to give a 4-amino-1-(2,6-dichloro-4-trifluoromethylphenyl)pyrazole.

Example 9:

3-Chloropyrazole is reacted with sodium hydride in DMF and then treated with 3-chloro-4-fluorobenzotrifluoride and heated to 110°C for 2 hr to give 3-chloro-1-(2-chloro-4-trifluoromethylphenyl)pyrazole.

Following the procedure of Example 7, the above phenylpyrazole (0.18 g, 0.64 mmol) is reacted with fuming nitric acid and fuming sulfuric acid to give 3-chloro-4-nitro-1-(2-chloro-4-trifluoromethylphenyl)pyrazole.

The above 3-chloro-4-nitro-1-(2-chloro-4-trifluoromethylphenyl)pyrazole is dissolved in 15 ml of methanol and treated with hydrochloric acid (3 ml, 5%) and stannous chloride (5.0 mmol) with heating under reflux for 8 hr. The reaction mixture is worked up by potassium hydroxide/water washes to give a mixture of 4-amino-3,5-dichloro-1-(2-chloro-4-trifluoromethylphenyl)pyrazole and 4-amino-5-chloro-3-methoxy-1-(2-chloro-4-trifluoromethylphenyl)pyrazole.

The above 3-chloro-4-nitro-1-(2-chloro-4-trifluoromethylphenyl)pyrazole is reduced by $H_2/PtO_2$ (cat.), following the procedure of Example 7, to give 4-amino-3-chloro-1-(2-chloro-4-trifluoromethylphenyl)pyrazole.

Example 10: 4-Amino-1-(2,4-dichlorophenyl)pyrazole

A mixture of 2,4-dichdlorophenylhydrazine hydrochloride (0.85 g, 4.0 mmol) and 1,1,3,3-tetramethoxypropane (0.72 g, 4.4 mmol) in 20 ml of ethanol is heated to reflux for 1 hr, and then concentrated in vacuo. The resulting product is dissolved in acetic anhydride (15 ml) and treated with 90% $HNO_3$ (0.7 ml), warmed to 80°C and then colled and poured into an ether/water mixture. The ether phase is neutralized (5% NaOH), washed with water and with brine, and dried. Flash chromatography gives 4-nitro-1-(2,4-dichlorophenyl)pyrazole. Reduction following Example 8 gives 4-amino-1-(2,4-dichloro-phenyl)pyrazole.

Example 11: 4-(Amino-1-(1,1,2,3,3,3-hexafluoropropyl)pyrazole

To a solution of 4-nitropyrazole (0.6 g, 5.3 mmol) and 5% potassium hydroxide (12 drops) in 15 ml of pyridine is bubbled 1,1,2,3,3,3-hexafluoropropene until a slow reflux (dry ice condenser) is obtained. After 1 hour the reaction mixture is cooled to 0° and an additional 6 drops of 5% potassium hydroxide is added. After 3.5 hours, the mixture is worked up by partition between water and ether/hexane. The organic layer is washed with 5% hydrochloric acid, water and 10% sodium bicarbonate, dried, concentrated and purified by flash chromatography to give 4-nitro-1-(1,1,2,3,3,3-hexafluoropropyl)pyrazole.

Following the procedure of Example 8, the above propylpyrazole is reduced by reaction with ethanol, platinum dioxide and hydrogen to give a 4-amino-1-(1,1,2,3,3,3-hexafluoropropyl)pyrazole.

**Claims**

1. A compound of the following formula (A)

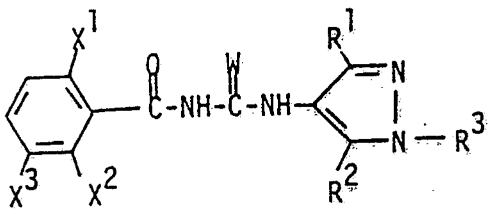 (A)

wherein W is oxygen or sulfur:

each of $X^1$, $X^2$ and $X^3$ is independently hydrogen, $C_{1-8}$alkyl or halogen;

each of $R^1$ and $R^2$ is independently hydrogen, $C_{1-8}$alkyl, $C_{1-8}$alkoxy, halogen or COOR;

R is hydrogen or $C_{1-8}$alkyl;

$R^3$ is unsubstituted or halogenated $C_{1-8}$alkyl, unsubstituted or halogenated $C_{2-8}$alkenyl, unsubstituted or halogenated $C_{3-8}$cycloalkyl, or the substituted phenyl group

$C_6H_{4-t}ZY_t$

wherein t is zero, one, two three or four; and

each of Y and Z is independently halogen, or unsubstituted or halogenated $C_{1-8}$alkyl.

2. A compound according to Claim I, wherein $X^3$, $R^1$ and $R^2$ are hydrogen, W is oxygen, $X^1$ is chloro or fluoro, $X^2$ is hydrogen or fluoro and $R^3$ is $C_6H_{4-t}ZY_t$.

3>. A compound according to Claim 2, wherein Y is chloro and Z is chloro or trifluoromethyl.

4. A compound according to Claim 3, wherein t is one or two.

5. A compound according to Claim 4, wherein each of $X^1$ and $X^2$ is fluoro.

6. The compound N-4-[I-(2-chloro-4-trifluoromethylphenyl)pyrazolyl]-N′-2,6-difluorobenzoylurea, according to Claim 5.

7. A compound according to Claim 4, wherein $X^1$ is chloro and $X^2$ is hydrogen.

8. Process of preparing compounds of formula (A) stated in Claim I, which comprises

a) reacting a benzoyl isocyanate or isothiocyanate of formula (I)

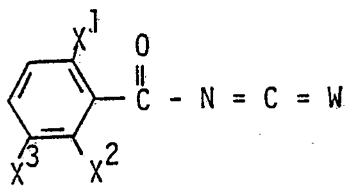 (I)

wherein $X^1$, $X^2$, $X^3$ and W are as defined in Claim I, with a 4-aminopyrazole of formula II

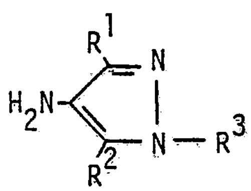 (II)

wherein $R^1$, $R^2$ and $R^3$ are as defined in Claim I, or

b) by reacting a benzamide of formula (III)

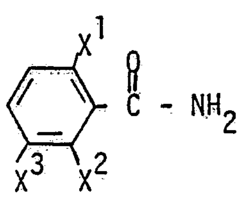 (III)

wherein $X^1$, $X^2$ and $X^3$ are as defined in Claim I,
with pyrazolyl isocyanate or isothiocyanate of formula (IV)

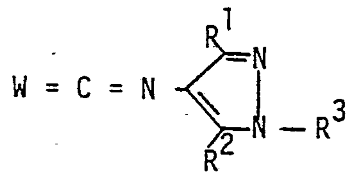

(IV)

wherein W, $R^1$, $R^2$ and $R_3$ are as defined in Claim I.

9. Pesticidal composition comprising a compound of formula (A) as defined in any one of Claims I to 7 and an agriculturally acceptable diluent.

I0. A method of combatting pests which comprises applying to the pest or its locus a pest-controlling amount of a compound of formula (A) as defined in any one of Claims I to 7.

Claims for the following Contracting States : AT, ES.

I. A pesticidal composition comprising a compound of the following formula (A)

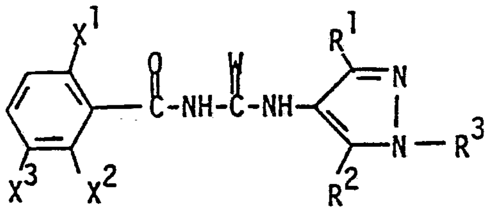

(A)

wherein W is oxygen or sulfur;

each of $X^1$, $X^2$ and $X^3$ is independently hydrogen, $C_{1-8}$alkyl or halogen;

each of $R^1$ and $R^2$ is independently hydrogen, $C_{1-8}$alkyl, $C_{1-8}$alkoxy, halogen or COOR;

R is hydrogen or $C_{1-8}$alkyl;

$R^3$ is unsubstituted or halogenated $C_{1-8}$alkyl, unsubstituted or halogenated $C_{2-8}$alkenyl, unsubstituted or halogenated $C_{3-8}$cycloalkyl, or the substituted phenyl group

$C_6H_{4-t}ZY_t$

wherein t is zero, one, two, three or four; and

each of Y and Z is independently halogen, or unsubstituted or halogenated $C_{1-8}$alkyl.

2. A composition according to Claim I, wherein $X^3$, $R^1$ and $R^2$ are hydrogen, W is oxygen, $X^1$ is chloro or fluoro; $X^2$ is hydrogen or fluoro and $R^3$ is $C_6H_{4-t}ZY_t$.

3. A composition according to Claim 2, wherein Y is chloro and Z is chloro or trifluoromethyl.

4. A composition according to Claim 3, wherein t is one or two.

5. A composition according to Claim 4, wherein each of $X^1$ and $X^2$ is fluoro.

6. The composition according to Claim 5 comprising N-4-[I-(2-chloro-4-trifluoromethylphenyl)pyrazolyl]-N'-2,6-difluorobenzoylurea.

7. A composition according to Claim 4, wherein $X^1$ is chloro and $X^2$ is hydrogen.

8. Process of preparing compounds of formula (A) stated in Claims I to 7, which comprises

a) reacting a benzoyl isocyanate or isothiocyanate of formula (I)

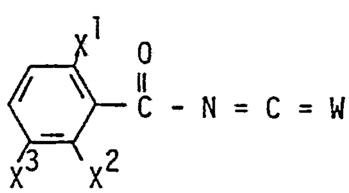

(I)

wherein $X^1$, $X^2$, $X^3$ and W are as defined in Claim I, with a 4-aminopyrazole of formula II

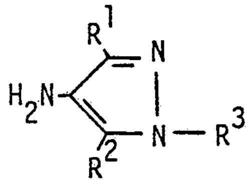

(II)

wherein $R^1$, $R^2$ and $R^3$ are as defined Claim I, or

b) by reacting a benzamide of formula (III)

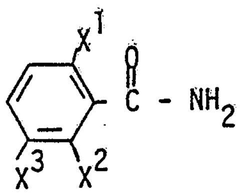

(III)

wherein $X^1$, $X^2$ and $X^3$ are as defined in Claim I,
with a pyrazolyl isocyanate or isothiocyanate of formula (IV)

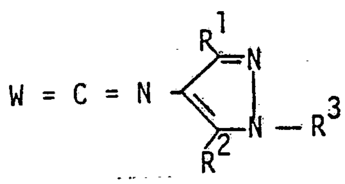

(IV)

wherein W, $R^1$, $R^2$ and $R_3$ are as defined in Claim I.